# FASCICULE DE BREVET EUROPEEN

(11) **EP 3 125 844 B1**
(45) Date de publication et mention de la délivrance du brevet: **24.07.2019**
(21) Numéro de dépôt: 15719794.8
(22) Date de dépôt: 02.04.2015
(51) Int. Cl.: A61F 13/62, A44B 18/00, A61F 13/15, B32B 37/00, B32B 5/06

(54) **PROCEDE D'ASSEMBLAGE D'AU MOINS DEUX ENSEMBLES ET STRUCTURE ASSEMBLEE CORRESPONDANTE**
VERFAHREN ZUM VERBINDEN MINDESTENS ZWEIER BAUGRUPPEN UND ENTSPRECHENDE VERBUNDSTRUKTUR
METHOD FOR JOINING AT LEAST TWO ASSEMBLIES AND CORRESPONDING JOINED STRUCTURE

(30) Priorité: 03.04.2014 FR 1452957
(43) Date de publication de la demande: 08.02.2017
(73) Titulaire: Aplix, 44850 Le Cellier (FR)
(72) Inventeur: BOSSER, Damien, Pierre, Antoine, F-44000 Nantes (FR)
(74) Mandataire: Gilbey, Vincent
(86) Numéro de dépôt international: PCT/FR2015/050867
(87) Numéro de publication internationale: WO 2015/150709

(56) Documents cités:
- EP-A1- 2 140 775
- EP-A1- 2 201 857
- WO-A1-98/10728
- GB-A- 299 825
- US-A- 3 935 360
- US-A- 5 798 163

## Description

### Domaine technique

Le présent exposé concerne un procédé pour l'assemblage de plusieurs ensembles.

### Arrière-plan du présent exposé

De nombreux produits sont issus de l'assemblage de plusieurs ensembles, notamment de plusieurs couches ou feuilles. C'est le cas notamment des courroies, qui sont généralement formées par l'assemblage, par chauffage, de deux bandes pleines et continues, séparées par une couche de fils de renfort.

Quelle que soit l'application, les différents ensembles sont positionnés relativement les uns par rapport aux autres avant d'être solidarisés. Plusieurs problèmes peuvent alors survenir. Les ensembles pré-positionnés peuvent être malencontreusement déplacés, avant ou pendant l'assemblage, entraînant un retard dans le processus de fabrication, ou la fabrication d'un produit défectueux qui devra être mis au rebut. Plus grave, le déplacement inopportun d'un ou des ensemble(s) peut être difficilement détectable lors de la fabrication, et entraîner un risque pour l'utilisateur du produit final.

Pour pallier ces problèmes et sécuriser le positionnement des différents ensembles avant et pendant assemblage, il a été envisagé d'utiliser des moyens de pré-assemblage tels que des pinces ou des agrafes. Ces moyens peuvent toutefois s'avérer difficiles à retirer, ce qui rend très compliqué le repositionnement en cas d'erreur. Ils peuvent créer, dans le produit, des zones de contraintes localisées. Ils peuvent aussi être impossibles à extraire, formant alors des corps étrangers dans le produit final.

Dans certaines applications, on a tenté d'utiliser des moyens adhésifs, moins intrusifs, pour le pré-assemblage. Dans le domaine des courroies par exemple, il est connu d'encoller les fils de renfort avec une composition adhésive, pour maintenir en position les deux bandes disposées de part et d'autre desdits fils.

Avec de tels moyens, il reste toujours difficile de corriger, après coup, un mauvais positionnement des ensembles. Ces moyens adhésifs peuvent être difficiles à fabriquer du fait des normes et réglementations environnementales à respecter. Ils présentent en outre l'inconvénient de perdre facilement leurs propriétés adhésives au fil du temps ou en conséquence de mauvaises conditions de stockage.

### Objet et résumé du présent exposé

L'un des objectifs du présent exposé est donc de fournir un procédé permettant de remédier aux inconvénients de l'art antérieur énoncés ci-dessus.

Cet objectif est atteint grâce à un procédé selon la revendication 1 dans lequel on fournit un premier ensemble comprenant un champ d'éléments agrippants, notamment des crochets, et un deuxième ensemble muni de moyens de retenue adaptés à coopérer avec les éléments agrippants du premier ensemble pour réaliser une fixation auto-agrippante ; on met le premier et le deuxième ensemble en contact de sorte que les éléments agrippants du premier ensemble et les moyens de retenue du deuxième ensemble réalisent une fixation auto-agrippante ; et on traite une zone de traitement de la fixation auto-agrippante pour déformer les éléments agrippants du premier ensemble et/ou les moyens de retenue du deuxième ensemble, ce par quoi le premier et le deuxième ensemble sont solidarisés définitivement et forment ainsi une structure assemblée.

Dans le procédé selon le présent exposé, le pré-positionnement d'un premier et d'un deuxième ensemble est sécurisé, préalablement à leur solidarisation définitive, au moyen d'une fixation auto-agrippante aussi appelée fermeture contact ou touch fastener en anglais. Une fixation auto-agrippante doit être entendue ici comme une liaison permettant d'immobiliser le premier et le deuxième ensemble l'un par rapport à l'autre dans au moins une direction, notamment une direction tangente et/ou orthogonale à la surface de jonction entre lesdits ensembles, surface de jonction pouvant être plane, courbe, ou présenter tout autre profil adapté.

Dans certains cas, notamment lorsque la fixation auto-agrippante immobilise le premier et le deuxième ensemble dans une direction orthogonale à la surface de jonction entre les deux ensembles, elle nécessite, pour être défaite, une force bien supérieure à la force appliquée aux ensembles lors de leur fixation.

Une fixation auto-agrippante peut ainsi assurer, sans effort, un maintien provisoire efficace d'au moins deux ensembles.

Si nécessaire, elle permet aussi leur détachement et leur repositionnement aisés et répétés, assurant toujours, in fine, un maintien du positionnement au moment de la solidarisation définitive.

Le pré-positionnement peut en outre être réalisé sans risque d'endommager les ensembles, aucun moyen d'attache ou outil extérieur n'étant nécessaire.

Dans le présent exposé, un élément agrippant peut présenter toute forme adaptée à coopérer avec des moyens de retenue complémentaires pour former une fermeture auto-agrippante du type défini ci-dessus, mâle-mâle, mâle-femelle ou hybride.

Un élément agrippant peut être crochet. Un crochet doit être entendu ici comme un élément adapté à s'accrocher (notamment à une boucle ou une fibre), en particulier un élément formé d'une tige et d'une partie d'accrochage surplombant ladite tige et s'étendant latéralement depuis celle-ci. Ainsi, un élément en forme de champignon, de harpon, un crochet à simple ou double aile d'accrochage ou analogue doit être entendu comme un crochet au sens de l'invention.

Un élément agrippant peut aussi se présenter sous la forme d'une simple tige.

De manière conventionnelle, ces éléments sont regroupés pour former un champ. Dans la présente demande, un champ d'éléments doit être entendu comme une pluralité d'éléments, notamment au moins 50 éléments, encore plus préférentiellement au moins 200 éléments, répartis régulièrement ou non.

La dimension des éléments agrippants et leur nombre par unité de surface (densité du champ d'éléments agrippants) peuvent varier sensiblement. Par exemple, les éléments agrippants présentent une hauteur totale, mesurée orthogonalement à la surface de la base dont ils sont issus, comprise entre 0,1 et 5 mm, de préférence entre 0,5 à 1,5 mm. De préférence, la densité du champ est comprise entre 1 et 2000 éléments/cm², de préférence 10 à 1200 éléments/cm².

Les moyens de retenue du deuxième ensemble peuvent également prendre des formes très diverses.

Dans la présente demande, des moyens de retenue peuvent comprendre un champ d'éléments agrippants.

Selon un autre exemple, les moyens de retenue comprennent des fibres. Dans la présente demande, une fibre doit être entendue comme un élément fin et allongé, continu ou discontinu, notamment une fibre ou un filament. Les fibres peuvent être assemblées pour former un non-tissé. Elles peuvent aussi être tissées. Elles peuvent encore être assemblées en faisceau, formant alors un câble. Ces fibres peuvent être des fibres synthétiques (de carbone, d'aramide ou de verre) ou encore des fibres naturelles (lin).

Selon un exemple, le deuxième ensemble peut ainsi comprendre une couche de non-tissé dont les fibres forment moyens de retenue. Comme variante, le deuxième ensemble peut aussi comprendre un tissu muni d'un champ de boucles formant moyens de retenue.

Pour solidariser définitivement le premier et le deuxième ensemble, la fixation auto-agrippante est traitée sur une zone de traitement de sorte que les éléments agrippants du premier ensemble et/ou les moyens de retenue du deuxième ensemble situés sur ladite zone de traitement sont déformés.

Le traitement de la fixation auto-agrippante peut être réalisé de différentes manières, envisagées comme des alternatives ou éventuellement en combinaison. Le traitement peut ainsi comprendre, de manière non exhaustive, l'application d'une pression, d'une vibration, d'une friction, d'un rayonnement sur la fixation auto-agrippante et/ou un traitement chimique utilisant au moins un solvant et/ou un traitement thermique, par exemple un chauffage par ultrasons (génération de vibrations ultrasoniques à l'aide d'une sonotrode notamment).

Selon un exemple de mise en oeuvre, le traitement est tel qu'après déformation, les éléments agrippants ou les moyens de retenue forment des moyens de renfort de la structure assemblée.

Dans la présente demande, un moyen de renfort d'une structure est un moyen conférant à ladite structure une partie substantielle (par exemple au moins 20%, de préférence au moins 30%, encore plus préférentiellement au moins 50%) de sa résistance à au moins un type de contrainte, notamment mécanique (par exemple résistance à la traction, à la compression, au cisaillement), thermique ou chimique.

Selon un exemple particulier, le traitement est tel qu'après déformation, les éléments agrippants ou les moyens de retenue conservent une forme globalement inchangée.

Par forme globalement inchangée, on entend une forme qui, bien qu'éventuellement modifiée par rapport à la forme d'origine, par ses dimensions ou sa structure, conserve les propriétés mécaniques de retenue de l'élément.

Dans ce cadre, une fibre peut éventuellement changer de diamètre, de longueur, ou de forme, mais reste globalement un moyen de retenue, notamment une fibre. Un crochet ou un harpon peut voir sa forme et/ou ses dimensions modifiées (préférentiellement, les dimensions caractéristiques de l'élément, notamment sa hauteur, sa largeur et/ou son épaisseur, varient d'au plus 20%, de préférence au plus 10%, encore plus préférentiellement au plus 5% par rapport à leur valeur initiale), mais reste globalement un élément agrippant. En se déformant, un crochet ou un harpon pourra, par exemple, devenir une tige.

Selon un exemple, dans le cas où le traitement est un traitement thermique, la fixation auto-agrippante est soumise à une température efficace supérieure à une première température de fusion de l'un seulement parmi les éléments agrippants et les moyens de retenue.

Selon un exemple, l'écart entre les températures de fusion des éléments agrippants et des moyens de retenue est par exemple au moins 5°C, préférentiellement au moins 10°C ou encore préférentiellement au moins 20°C.

Il est courant qu'une partie d'un produit nécessite d'être renforcée, pour améliorer sa résistance mécanique, thermique ou chimique. Ce renforcement peut par exemple résulter de l'inclusion, dans le produit, de moyens de renfort. Grâce aux dispositions précitées, le procédé selon le présent exposé permet de renforcer un produit en y intégrant des moyens de renfort pouvant prendre la forme de fibres ou d'éléments agrippants.

Grâce à la fixation auto-agrippante réalisée au préalable entre le premier et le deuxième ensemble, les moyens de renfort sont maintenus en position avant et pendant le traitement. Leur bon positionnement au sein de la structure finale assemblée est donc assuré, garantissant un renfort optimal. Dans le cas où les moyens de renfort sont constitués par des éléments agrippants, on évite par exemple que ces éléments soient inclinés ou repliés sur eux-mêmes au moment du traitement, ce qui nuirait à l'effet de renforcement. Dans le cas où les moyens de renfort sont constitués par des fibres, on évite notamment que les fibres s'éloignent excessivement de l'ensemble à renforcer ou qu'elles ne se répartissent pas de façon homogène sur cet ensemble.

Le procédé selon le présent exposé peut ainsi être utilisé pour la fabrication d'un élément composite dans lequel les moyens de retenue de l'un parmi le premier ou le deuxième ensemble forment moyens de renfort et les moyens de retenue de l'autre ensemble forment une matrice dans laquelle sont noyés lesdits moyens de renfort.

Selon un exemple, la zone de traitement forme une ligne continue.

Elle s'étend par exemple sur une longueur d'au moins 1 centimètre, de préférence au moins 3 centimètres.

Selon un autre exemple, la zone de traitement peut aussi être discontinue, notamment formée par un ensemble de points.

De préférence, la zone de traitement représente au moins 50%, de préférence au moins 80%, en particulier au moins 95%, de l'étendue totale de la fixation auto-agrippante.

Dans un mode de mise en oeuvre particulièrement préféré, la zone de traitement recouvre l'ensemble de la fixation auto-agrippante. Encore plus préférentiellement, elle recouvre toute la surface du champ d'éléments agrippants et/ou des moyens de retenue.

Selon un exemple, la fixation auto-agrippante recouvre au moins 50%, de préférence au moins 80%, en particulier au moins 95%, de l'étendue de la zone de contact entre le premier et le deuxième ensemble.

Selon un exemple, le premier et le deuxième ensemble peuvent être traités au-delà de la fixation auto-agrippante. Ces ensembles peuvent, en particulier, être traités dans leur intégralité.

Les éléments agrippants et/ou les moyens de retenue sont réalisés dans un matériau thermoplastique.

Les éléments agrippants et/ou les moyens de retenue peuvent ainsi être réalisés, de manière non limitative, dans l'un des matériaux suivants : polyéthylène, polypropylène ou autre homopolymère ou copolymère d'oléfine, comme les éthylènes/alpha oléfines commercialisées sous les appellations Affinity®, Engage® ou Exact® ou les polyoléfines semi cristallines commercialisées sous les appellations Vistamaxx® ou Versify® ou les thermoplastiques élastomères commercialisés sous les appellations Santoprène®, Sofprene® ou Thermolast® ; polyuréthane de type polyester, polyéther, polycarbonate, notamment les polyesters comme le PET, le PBT, le PTT, le PETG, la PCL, le PLA, les copolymères à base de polyester tels que les HYTREL® et les ARNITEL®; les polyamides comme par exemple le PA6, le PA 6.6, le PA 11 et le PA12, les copolymères à base de polyamide comme tels que les PEBAX® et les VESTAMID®, les POM homo ou copolymère, les alliages contenant au moins un des polymères précités.

Selon un exemple, les éléments agrippants et/ou les moyens de retenue comprennent des particules métalliques, généralement noyées dans le matériau thermoplastique qui les constitue. Ces particules métalliques permettent, lors d'un traitement thermique notamment, que la fixation auto-agrippante soit chauffée plus rapidement et de manière plus importante localement, améliorant ainsi son traitement, par exemple par induction.

Le procédé selon le présent exposé peut aussi permettre d'assembler plus de deux ensembles. Selon un exemple de mise en oeuvre, on fournit un troisième ensemble comprenant un champ d'éléments agrippants adaptés à coopérer avec des moyens de retenue du deuxième ensemble pour réaliser une fixation auto-agrippante ; on met le deuxième et le troisième ensemble en contact de sorte que le deuxième ensemble est disposé entre le premier et le troisième ensemble, et les éléments agrippants du troisième ensemble et les moyens de retenue du deuxième ensemble réalisent une deuxième fixation auto-agrippante ; et on traite une zone de traitement de la deuxième fixation auto-agrippante pour déformer les éléments agrippants du troisième ensemble et/ou les moyens de retenue du deuxième ensemble, ce par quoi le deuxième et le troisième ensemble sont solidarisés définitivement.

De préférence, les fixations auto-agrippantes réalisées entre le deuxième ensemble et respectivement le premier et le troisième ensemble sont traitées concomitamment en une seule et même étape de traitement.

Selon un exemple particulier de mise en oeuvre, préalablement au traitement, les éléments agrippants du premier ensemble et les éléments agrippants du troisième ensemble sont amenés en coopération, les moyens de retenue du deuxième ensemble étant compris entre lesdits éléments agrippants.

Selon un exemple, les ensembles sont assemblés en continu, sur une ligne de production.

Dans ce cas, par exemple, le premier et le deuxième ensemble forment chacun une bande s'étendant longitudinalement, le premier et le deuxième ensemble sont superposés continûment (en ligne) dans une direction longitudinale sur au moins une zone de contact sur laquelle les éléments agrippants du premier ensemble et les moyens de retenue du deuxième ensemble réalisent une fixation auto-agrippante, et ladite fixation auto-agrippante est traitée continûment (en ligne) dans la direction longitudinale sur au moins une zone de traitement. La zone de traitement peut être continue ou discontinue. Le traitement est par exemple réalisé au moyen d'une molette réalisant un contact continu (dans ce cas la zone de traitement comprend au moins une bande continue) ou des contacts ponctuels (dans ce cas la zone de traitement comprend plusieurs zones distinctes espacées dans la direction longitudinale).

Le procédé selon le présent exposé peut avoir des applications diverses, notamment dans le domaine de l'hygiène.

Selon un exemple d'utilisation, le procédé peut servir à la fabrication de porte-crochets pour couches culottes.

Dans ce cas, notamment, le premier ensemble comporte un champ de crochets et le deuxième ensemble est un non-tissé, le premier et le deuxième ensemble sont superposés sur au moins une zone de contact sur laquelle les crochets du premier ensemble et les fibres du deuxième ensemble réalisent une fixation auto-agrippante tout en préservant de part et d'autre de la zone de contact une portion libre du premier ensemble présentant des crochets et une portion libre du deuxième ensemble, et la fixation auto-agrippante est traitée pour solidariser le premier et le deuxième ensemble.

A noter que l'on entend ici par portion libre d'un ensemble une portion qui n'est pas en contact avec l'autre parmi le premier et le deuxième ensemble, et dont les moyens de retenue sont généralement (mais non nécessairement) opérationnels i.e. aptes à coopérer avec d'autres moyens pour former une fixation auto-agrippante.

Selon un deuxième exemple d'utilisation, le procédé selon le présent exposé peut servir au renforcement d'une base, notamment une nappe ou un élément composite, au moyen d'au moins un insert de renfort du type oeillet, à un emplacement de perforation de ladite base. Dans ce cas, l'insert de renfort constitue généralement le premier ensemble et la base, le deuxième ensemble.

Par emplacement de perforation, on entend ici un emplacement où la base est - ou est destinée à être - perforée.

Le présent exposé concerne également une structure selon la revendication 13 comprenant au moins une première couche comportant des moyens de retenue pour une fixation auto-agrippante et au moins une deuxième couche coopérant avec la première couche sur au moins une zone d'assemblage en emprisonnant les moyens de retenue, ce par quoi la première et la deuxième couche sont définitivement solidarisées l'une à l'autre.

Les moyens de retenue peuvent comprendre des fibres. Plus particulièrement, la première couche peut être constituée d'une nappe de fibres, tissées ou non-tissées.

Selon un autre exemple, les moyens de retenue sont des éléments agrippants, notamment des crochets. Par exemple, la première couche comporte une base présentant au moins une face dont fait saillie un champ d'éléments agrippants.

Selon un exemple, une structure selon le présent exposé peut être un porte-crochets pour couche-culotte. Un tel porte-crochets comprend une couche de base munie d'une pluralité de crochets et une couche de fibres, généralement du non-tissé, reliées au niveau d'une zone d'assemblage.

Selon encore un autre exemple, une structure selon le présent exposé comprend une base et un insert de renfort muni d'un trou traversant, fixé à un emplacement de perforation de ladite base.

Généralement, le trou de l'insert est disposé en regard de l'emplacement de perforation de la base.

La base peut par exemple être une nappe, notamment une bâche, par exemple formée par une couche de non-tissé, ou encore un élément composite, par exemple un panneau de garniture intérieur de véhicule.

Plusieurs exemples de mise en oeuvre sont décrits dans le présent exposé. Toutefois, sauf précision contraire, les caractéristiques décrites en liaison avec un exemple de mise en oeuvre quelconque peuvent être appliquées à un autre exemple de mise en oeuvre.

### Brève description des dessins

Le présent exposé sera bien compris et ses avantages apparaîtront mieux, à la lecture de la description détaillée qui suit, de plusieurs modes de mise en oeuvre représentés à titre d'exemples non limitatifs. La description se réfère aux dessins annexés sur lesquels :
- La figure 1 montre, en perspective, deux ensembles destinés à être assemblés par un premier exemple de mise en oeuvre du procédé selon le présent exposé ;
- La figure 2A est une vue de côté des deux ensembles de la figure 1, avant mise en contact ;
- La figure 2B est une vue de côté des deux ensembles de la figure 1, liés par une fixation auto-agrippante ;
- La figure 2C est une vue de côté de la structure résultant de l'assemblage définitif des premier et deuxième ensembles de la figure 1 ;
- La figure 3 illustre schématiquement la zone de traitement de la fixation auto-agrippante de la figure 2B ;
- Les figures 4A à 4C illustrent des variantes de répartition du traitement de la fixation auto-agrippante de la figure 2B ;
- La figure 5A est une section montrant trois ensembles destinés à être assemblés par un deuxième exemple de mise en oeuvre du procédé selon le présent exposé, avant mise en contact ;
- La figure 5B est une section montrant les trois ensembles de la figure 5A, liés par une fixation auto-agrippante ;
- La figure 5C est une section de la structure résultant de l'assemblage définitif des trois ensembles de la figure 5A ;
- La figure 6 illustre une variante du deuxième exemple de mise en oeuvre du procédé selon le présent exposé ;
- Les figures 7A à 7C illustrent les étapes successives d'un troisième exemple de mise en oeuvre du procédé selon le présent exposé ;
- La figure 8 illustre une première variante du troisième exemple de mise en oeuvre du procédé selon le présent exposé ;
- La figure 9 illustre une deuxième variante du troisième exemple de mise en oeuvre du procédé selon le présent exposé ;
- La figure 10 est une vue d'ensemble d'une couche-culotte d'usage courant ;
- La figure 11 illustre une ligne de fabrication de porte-crochets pour couches culottes par la mise en oeuvre du procédé selon le présent exposé;
- Les figures 12A et 12B sont des sections respectivement selon XIIA-XIIA et XIIB-XIIB de la figure 11 ;
- La figure 13 illustre une base destinée à être renforcée localement par un insert de renfort ;
- Les figures 14A à 14D sont des sections selon XIV de la figure 13, montrant les différentes étapes d'assemblage de l'insert de renfort ;
- Les figures 15A et 15B illustrent une variante de réalisation du procédé d'assemblage des figures 13 et 14A à 14D ;
- La figure 16 illustre une variante du procédé de renforcement des figures 13 et 14A à 14D.

### Description détaillée d'exemples de réalisation

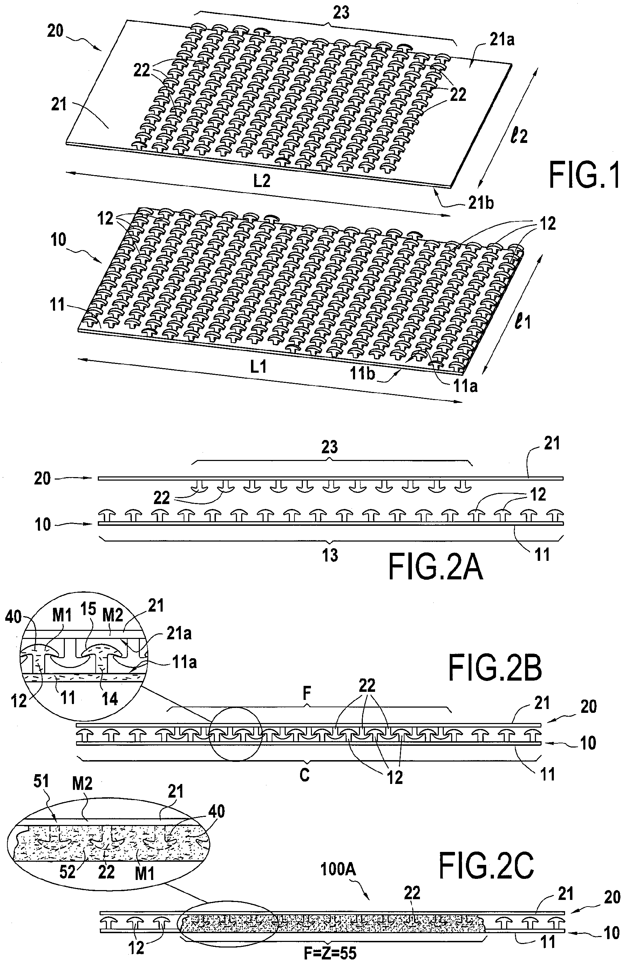

Les figures 1 à 3 illustrent la réalisation d'une structure 100A (voir figure 2C) par assemblage d'un premier ensemble 10 et d'un deuxième ensemble 20, selon un premier exemple de mise en oeuvre du procédé selon le présent exposé.

Le premier ensemble 10 est réalisé dans un premier matériau thermoplastique M1, fusible à une température T1, notamment un polymère réticulable tel que le polyéthylène pour lequel T1 est égal à 120°C. Dans l'exemple particulier des figures 1 à 3, le premier matériau M1 renferme en outre une pluralité de particules métalliques 40, dont la fonction sera détaillée dans la suite de la présente description.

Comme illustré sur la figure 1, le premier ensemble 10 comporte une base 11 délimitée par deux surfaces principales 11a, 11b, ici rectangulaires, sensiblement planes et parallèles, de longueur L1 et largeur l1.

Une pluralité d'éléments agrippants 12 fait saillie depuis l'une de ces surfaces principales 11a (ci-après surface de jonction), formant un champ d'éléments agrippants 13. Les éléments agrippants 12 sont par exemple moulés par injection en même temps que la base 11. Ils sont ainsi d'une seule pièce avec la base 11, autrement dit d'un seul tenant, sans interface ni discontinuité à leur jonction avec la base 11, notamment au niveau microscopique.

Les éléments agrippants 12 sont ici des crochets comportant chacun une tige 14 faisant saillie depuis la surface de jonction dans une direction sensiblement orthogonale à cette surface, et une partie d'accrochage 15, surplombant ladite tige 14 et comportant deux ailes d'accrochage s'étendant latéralement depuis la tige, de part et d'autre de celle-ci.

Dans l'exemple, le champ de crochets 13 s'étend sur toute la largeur Il et toute la longueur L1 de la base 11. Bien évidemment, cette répartition peut être très différente selon les applications envisagée. Les éléments agrippants peuvent ne recouvrir qu'une partie de la surface du premier ensemble dont ils sont issus, par exemple au moins 95%, ou encore moins de 5%.

Les éléments agrippants 12 présentent avantageusement une hauteur totale, mesurée orthogonalement à la surface 11a de la base 11, comprise entre 0,1 et 5 mm, et la densité du champ est comprise entre 1 et 2000 éléments/cm².

Le deuxième ensemble 20 est réalisé dans un deuxième matériau thermoplastique M2, fusible à une température T2 supérieure à T1, notamment un polymère tel que le polyamide pour lequel T2 est égale à 260°C.

Le deuxième ensemble 20 présente une structure très similaire à celle du premier ensemble 10, et n'est donc pas détaillé dans la suite. Il comporte, lui-aussi, une base 21 délimitée par deux surfaces 21a, 21b ici rectangulaires sensiblement planes et parallèles, de longueur L2 (ici identique à L1) et de largeur l2 (ici identique à l1).

Comme illustré sur la figure 1, le deuxième ensemble 20 comporte des moyens de retenue 22 adaptés à coopérer avec les éléments agrippants 12 du premier ensemble 10.

Dans l'exemple, ces moyens de retenue 22 sont des éléments agrippants complémentaires des crochets 12, en particulier des crochets de même forme, formant un champ de crochets 23.

Le champ de crochets 23 du deuxième ensemble 20 occupe ici une partie seulement de la surface de jonction 21a dont il est issu.

Lors de l'assemblage, comme illustré sur la figure 2A, le premier et le deuxième ensemble 10, 20 sont positionnés de sorte que leurs surfaces de jonction respectives 11a, 21a sont en regard l'une de l'autre.

Comme illustré sur la figure 2B, le premier et le deuxième ensemble 10, 20 sont ensuite amenés en contact, dans une position relative souhaitée.

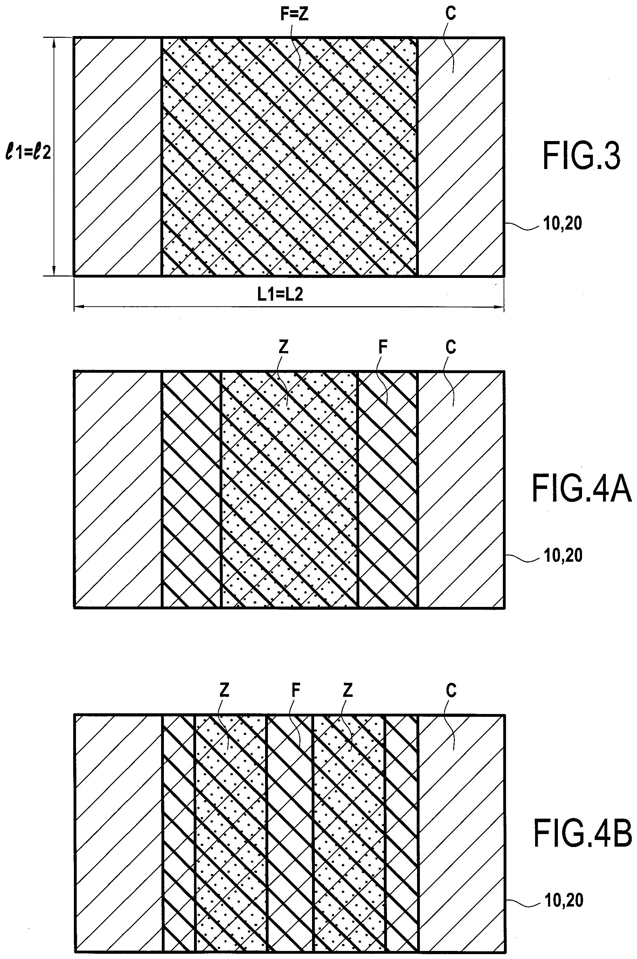

Sur les figures 2B et 3 notamment, on note C la zone de contact entre le premier et le deuxième ensemble 10, 20. Au niveau de cette zone de contact C, les champs de crochets 13, 23 du premier et du deuxième ensemble 10, 20 se font face sur au moins une zone déterminée, où ils réalisent, par coopération de leurs éléments agrippants 12, 22, une fixation auto-agrippante notée F.

Dans cet état, le premier et le deuxième ensemble 10, 20 sont immobilisés l'un par rapport à l'autre au niveau de ladite fixation auto-agrippante F, dans une direction orthogonale à leurs surfaces de jonction 11a, 21a et dans des directions tangentielles à ces surfaces. Cette immobilisation n'est cependant pas définitive et les deux ensembles 10, 20 peuvent facilement être détachés pour être repositionnés, si nécessaire.

Enfin, et comme représenté sur la figure 2C, la fixation auto-agrippante F est traitée sur une zone de traitement (notée Z et illustrée en pointillés, sur la figure 3) correspondant ici à l'intégralité de la zone de la fixation auto-agrippante F, de façon à solidariser définitivement le premier et le deuxième ensemble 10, 20.

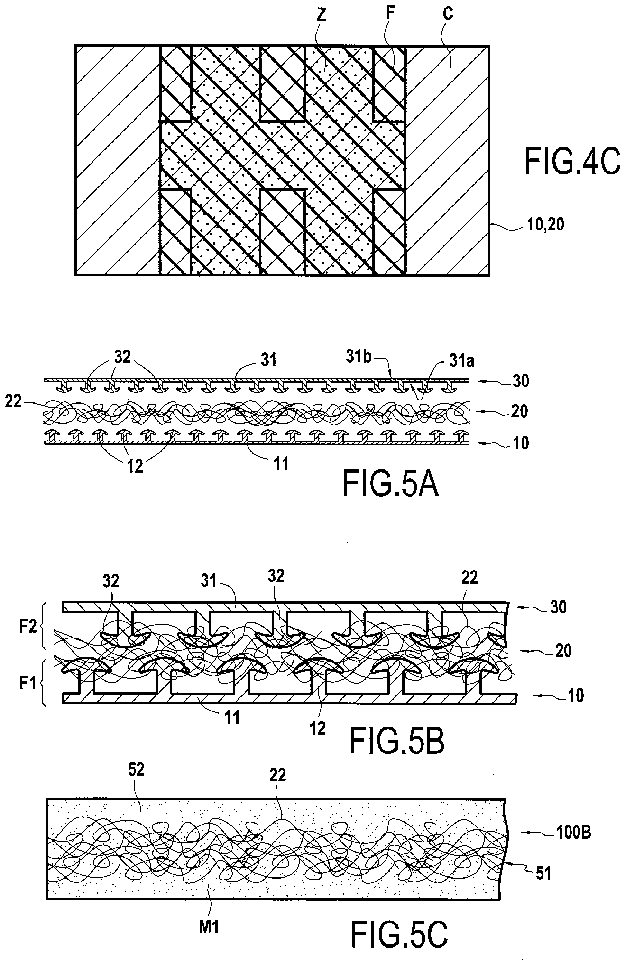

Cet exemple n'est cependant pas limitatif. La zone de traitement Z peut, dans certains cas, s'étendre sur une partie seulement de la fixation auto-agrippante F. Dans l'exemple de la figure 4A, la zone de traitement Z prend ainsi la forme d'une bande s'étendant sur une partie seulement de la longueur de la fixation auto-agrippante F, mais traversant le premier et le deuxième ensemble 10, 20 de part en part selon une ligne continue, ici dans le sens de la largeur. Dans les exemples des figures 4B et 4C, la zone de traitement Z comprend plusieurs bandes, s'étendant parallèlement les unes aux autres ou formant un quadrillage. Selon d'autres variantes, la zone de traitement peut aussi se présenter sous forme d'une pluralité de points ou équivalent.

Dans l'exemple des figures 1 à 3, la zone de traitement Z est soumise à une température T supérieure à T1 mais inférieure à T2, entraînant la déformation du premier ensemble sur ladite zone.

Les particules métalliques 40, dont on comprend qu'elles sont optionnelles, permettent ici d'augmenter rapidement et de façon homogène la température au sein du premier matériau.

Comme illustré sur la figure 2C, la base 11 et les crochets 12 du premier élément 10 fondent, et viennent enrober les crochets 22 du deuxième ensemble 20, restés intacts (car non déformés).

Pour assurer un traitement efficace, évitant la déformation des crochets 22 du deuxième élément, l'écart entre les températures de fusion respectives T1 et T2 du premier et du deuxième matériau M1, M2 est au moins égale à 5°C, préférentiellement au moins égale à 10°C, plus particulièrement au moins égale à 20°C.

La structure assemblée 100A ainsi obtenue est un bloc d'un seul tenant comprenant une première couche 51 comportant des moyens de retenue pour une fixation auto-agrippante, ici des crochets 22, et une deuxième couche 52 coopérant avec la première couche 51 sur au moins une zone d'assemblage 55 en emprisonnant les moyens de retenue 22, ce par quoi la première et la deuxième couche 51, 52 sont définitivement solidarisées l'une à l'autre.

La structure 100A forme donc un élément composite dont la matrice est formée par le matériau de la deuxième couche (résultant de la déformation du premier ensemble) et les moyens de renfort sont formés par les éléments agrippants de la première couche 51, améliorant ici notamment la résistance au cisaillement et/ou en traction de la structure.

A noter que, dans une variante, la température de fusion du premier ensemble peut aussi être supérieure à celle du deuxième ensemble.

Sur les figures 5A à 5C, on a illustré la réalisation d'une structure 100B (voir la figure) par assemblage d'un premier ensemble 10, d'un deuxième 20 et d'un troisième ensemble 30, selon un deuxième exemple de mise en oeuvre du procédé selon le présent exposé.

Comme illustré sur la figure 5A, le premier ensemble 10 est similaire à celui décrit en liaison avec la figure 1 notamment. Il n'est donc pas décrit une nouvelle fois.

Dans cet exemple, le deuxième ensemble 20 est constitué d'une couche de matériau non-tissé, réalisé dans un matériau thermoplastique fusible à une température T2, supérieure à T1. Les moyens de retenue 22 de ce deuxième ensemble 20, adaptés à coopérer avec les crochets 12 du premier ensemble 10, sont formés par les fibres entremêlées constituant le non-tissé.

Un non-tissé est constitué d'une pluralité de fibres liées entre elles et formant généralement une nappe. La liaison entre les fibres est réalisée mécaniquement, chimiquement ou thermiquement. On connaît actuellement trois grands types de non tissés : les Dry-laid Nonwovens (Carded thermobonded - Airlaid thermobonded - Spunlace - airthrough - Carded needle punched, etc.), les « Wet-laid Nonwovens », et les « Spunmelt Nonwovens» (Spunbond, Melblown ou une combinaison des deux (SM, SMS, SMMS, SSMMS, ...), Electrospun, Melt-film fibrillated, Solvent-spun, ...).

Le troisième ensemble 30 est réalisé dans un matériau polymère fusible, ici identique au matériau M1 constituant le premier ensemble 10.

Sa structure est, de la même manière, similaire à celle du premier ensemble 10. Il comporte ainsi une base 31 délimitée par deux surfaces sensiblement planes et parallèles 31a, 31b et une pluralité d'éléments agrippants 32 adaptés à coopérer avec les moyens de retenue 22 du deuxième ensemble 20. Dans l'exemple, ces éléments agrippants 32 forment un champ de crochets 33 faisant saillie depuis l'une des surfaces 31a de la base 31 (ci-après surface de jonction).

Lors de l'assemblage, et comme illustré sur la figure 5B, le premier et le deuxième ensemble 10, 20 sont amenés en contact, dans une position souhaitée. Les fibres 22 du deuxième ensemble et les crochets 12 du premier ensemble 10 coopèrent pour réaliser une première fixation auto-agrippante F1.

De la même manière, le deuxième ensemble 20 et le troisième ensemble 30 sont amenés en contact, dans une position souhaitée. Les fibres 22 du deuxième ensemble 20 et les crochets 12 du troisième ensemble 30 coopèrent pour réaliser une seconde fixation auto-agrippante F2.

Grâce à ces fixations auto-agrippantes F1, F2, les trois ensembles 10, 20, 30 sont maintenus en position les uns par rapport aux autres. En cas de mauvais positionnement, ils peuvent aisément être détachés, puis repositionnés, et ce, un grand nombre de fois, sans pour autant être endommagés.

Dans l'exemple, l'assemblage définitif des trois ensembles précités 10, 20, 30 est réalisé par traitement thermique, consistant à soumettre l'empilement à une température T, inférieure à T2 mais supérieure à T1.

Sous l'effet de la chaleur, le premier et le troisième ensemble 10, 30 se déforment, se lient l'un à l'autre, et viennent emprisonner les fibres 22 du non-tissé qui, elles, restent sensiblement intactes, le premier, deuxième et troisième ensemble étant ainsi définitivement solidarisés l'un à l'autre.

La structure assemblée 100B ainsi obtenue, illustrée sur la figure 5C, est un ensemble composite comprenant une première couche 51 comportant des moyens de retenue ayant été utilisés dans une fixation auto-agrippante, ici des fibres 22, et une deuxième couche 52 résultant de la déformation du premier et du troisième ensemble, coopérant avec la première couche 51 sur au moins une zone d'assemblage en emprisonnant les moyens de retenue 22, ce par quoi la première et la deuxième couche 51, 52 sont définitivement solidarisées l'une à l'autre.

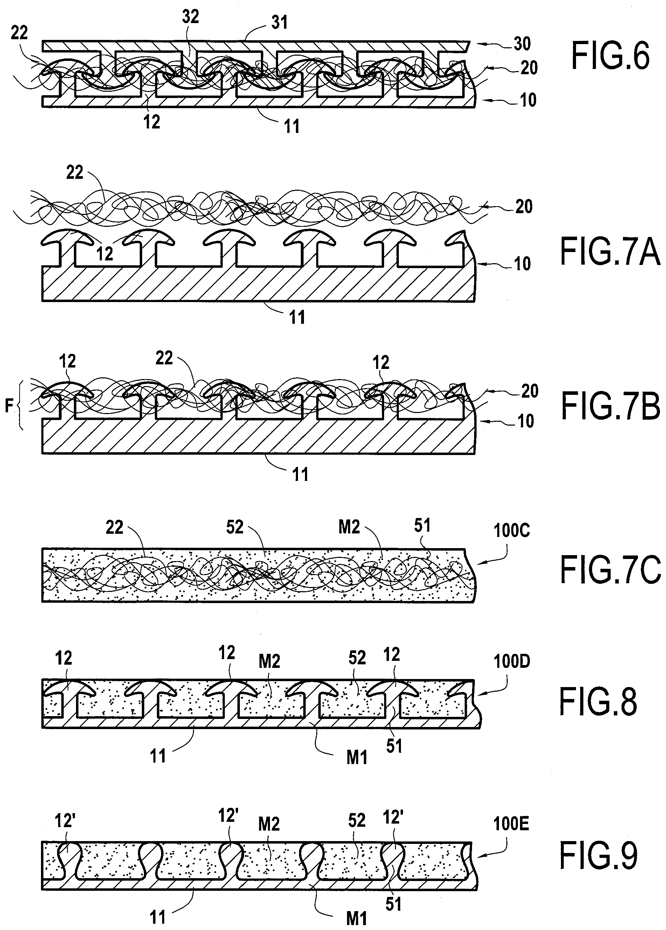

Les fibres 22 forment des moyens de renfort de la structure assemblée 100B, augmentant notamment sa rigidité et sa résistance à la traction, et évitant le décollement du premier et du troisième ensemble 10, 30. Selon une variante de mise en oeuvre illustrée sur la figure 6, le positionnement des trois ensembles 10, 20, 30 peut encore être sécurisé d'une façon complémentaire en amenant les crochets 12 du premier ensemble 10 et ceux 32 du troisième ensemble 30 en coopération, préalablement au traitement.

Il résulte de ce qui précède que le procédé selon le présent exposé peut avantageusement être utilisé dans le but de former une structure renforcée. Un troisième exemple de mise en oeuvre du procédé selon le présent exposé, illustrant une telle utilisation, est représenté schématiquement sur les figures 7A à 7C.

Dans cet exemple, un premier ensemble à renforcer 10, réalisé dans un matériau polymère fusible à une température T1, est sensiblement identique au premier ensemble décrit précédemment en liaison avec la figure 1 notamment.

Un deuxième ensemble 20 formé par une couche de non-tissé constituée de fibres enchevêtrées 22 (voir la figure 7A), est amené en contact avec la surface de jonction 11a du premier ensemble 10 dont font saillie ses éléments agrippants 12, de sorte que lesdites fibres 22 coopèrent avec les crochets 12 du premier ensemble 10 pour réaliser une fixation auto-agrippante F.

Dans cette position illustrée sur la figure 7B, la couche de non-tissé est immobilisée par rapport au premier ensemble dans des directions tangentes à la surface de jonction 11a et dans une direction orthogonale à cette surface.

La fixation auto-agrippante F est ensuite soumise à une température T supérieure à T1 mais inférieure à T2, obtenue par exemple par génération de vibrations ultrasoniques à l'aide d'une sonotrode. Dans cette position, la base 11 et les crochets 12 du premier ensemble fondent, et viennent enrober les fibres 22 du non-tissé, restées sensiblement intactes.

Le premier et le deuxième ensemble 10, 20 constituent alors une structure assemblée monobloc 100C sous forme de composite, illustré sur la figure 7C, comprenant une couche formant une matrice 52 résultant de la déformation du premier ensemble, emprisonnant des moyens de retenue pour une fixation auto-agrippante, ici des fibres 22.

Selon une première variante de ce troisième exemple de mise en oeuvre, illustrée sur la figure 8, le premier ensemble 10 est réalisé dans un matériau fusible à une température T2 supérieure à la température de fusion T1 du deuxième ensemble 20.

Lorsque la fixation auto-agrippante F réalisée entre les deux ensembles est traitée en étant soumise à une température intermédiaire, supérieure à T1 mais inférieure à T2, les fibres 22 fondent, et viennent enrober les crochets 12, restés, eux, intacts.

Le premier et le deuxième ensemble 10, 20 constituent alors une structure monobloc 100D sous forme de composite, comprenant une matrice (résultant de la déformation du deuxième ensemble 20) renforcée par les crochets 12 du premier ensemble 10.

Selon une deuxième variante de mise en oeuvre illustrée sur la figure 9, la fixation auto-agrippante F est traitée en étant soumise à une température supérieure aux températures de fusion du premier et du deuxième ensemble 10, 20.

Dans ce cas, sous l'effet de la chaleur, les crochets 12 fondent tout en conservant cependant une forme globale sensiblement cylindrique.

Les fibres 22, elles, fondent et viennent enrober les crochets déformés 12'.

Le procédé selon le présent exposé trouve des applications très diverses dans de nombreux domaines. Des exemples non exhaustifs en sont donnés ci-dessous.

Le procédé selon le présent exposé peut par exemple être utilisé avantageusement dans le domaine de l'hygiène, en particulier pour la fabrication de porte-crochets pour la fermeture de couches culottes.

Le procédé selon le présent exposé peut aussi être utilisé dans le domaine de l'automobile ou de l'aéronautique, notamment pour la fixation de garnitures de portes, de pavillons ou de capots.

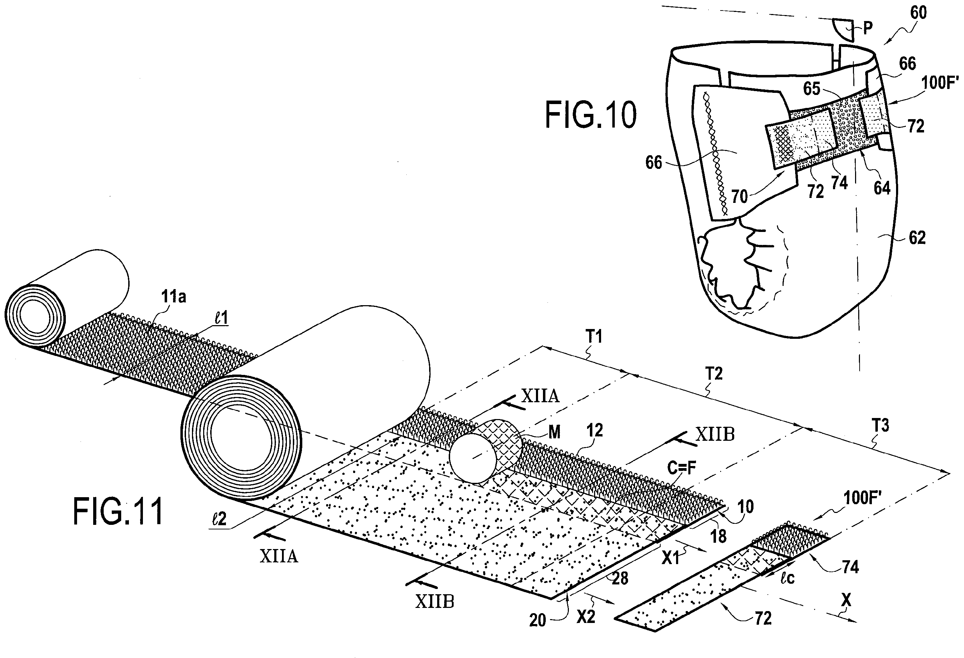

Les figures 10, 11, 12A et 12B illustrent une application particulière du procédé selon le présent exposé, pour la fabrication de porte-crochets pour couche-culotte.

Une couche-culotte 60 telle qu'illustrée sur la figure 10 comprend usuellement :
- une partie principale ou partie culotte 62 dont la face interne est destinée à venir en contact avec la peau du bébé et présentant généralement une partie interne absorbante et une partie externe imperméable,
- une bande frontale 64 centrée sur un plan de symétrie P de la couche, fixée à l'avant de la partie culotte 62 et présentant sur sa surface externe des fibres 65, notamment des boucles, destinées à coopérer avec des crochets auto-agrippants,
- deux oreilles 66, généralement élastiques, fixées à l'arrière de la partie culotte 62 (de part et d'autre du plan de symétrie P de la couche 60), et
- deux porte-crochets 100F' munis de crochets auto-agrippants destinés à venir coopérer avec les boucles de la bande frontale pour fermer la couche 60 (comme illustré sur la figure 10), chaque porte-crochets 100F' étant fixé sur une oreille 66.

Comme représenté sur la figure 10, un porte-crochets 100F' comprend généralement une partie support 72 comprenant des fibres (généralement en non-tissé) et qui est fixée à l'oreille 66 (généralement par soudure), et une partie de fixation 74 munie sur une face avant de crochets auto-agrippants 12 destinés à venir coopérer avec les fibres formant boucles de la bande frontale 64 de la couche 60, pour la fermeture de ladite couche.

De manière connue, ces deux parties 10, 20 peuvent être assemblées en soudant ou collant la face arrière d'un ruban dépourvue de crochets, sur un ruban de non-tissé. Ces méthodes présentent l'inconvénient de nécessiter des moyens précis et sûrs de guidage des rubans pour assurer leur pré-positionnement avant soudure ou au moment du collage. Par ailleurs, la résistance à la traction des porte-crochets ainsi obtenus est parfois insuffisante, entraînant un décollement ou un désassemblage des parties support et de fixation.

Le procédé d'assemblage selon le présent exposé permet de résoudre les problèmes précités.

La figure 11 illustre l'application de ce procédé sur une ligne de fabrication de porte-crochets.

Un premier ensemble 10 se présente ici sous la forme d'un ruban de fixation de largeur 11, s'étendant dans une direction longitudinale X1, et dont une face de jonction 11a est recouverte d'un champ de crochets 13, et un deuxième ensemble 20 est constitué d'un ruban support réalisé en non-tissé, de largeur 12 et s'étendant dans une direction longitudinale X2.

Dans une première étape illustrée par le tronçon noté T1 sur la figure 11, le premier et le deuxième ruban 10, 20 sont disposés parallèlement l'un à l'autre et superposés continument dans une direction longitudinale ou direction machine X sur une zone de contact C de largeur lc, le ruban support 20 recouvrant ainsi, en partie, la face de jonction 11a du ruban de fixation 10.

Sur la zone de contact C, les crochets 12 du ruban de fixation 10 et les fibres 22 du ruban support 20 réalisent une fixation auto-agrippante F. Dans l'exemple, cette fixation F s'étend sur l'ensemble de ladite zone de contact C.

La superposition des deux rubans 10, 20 est telle qu'une portion libre 18 du ruban de fixation 10, portant des crochets 12, et une portion libre 28 du ruban support 20, sont préservées de part et d'autre de la zone de contact C.

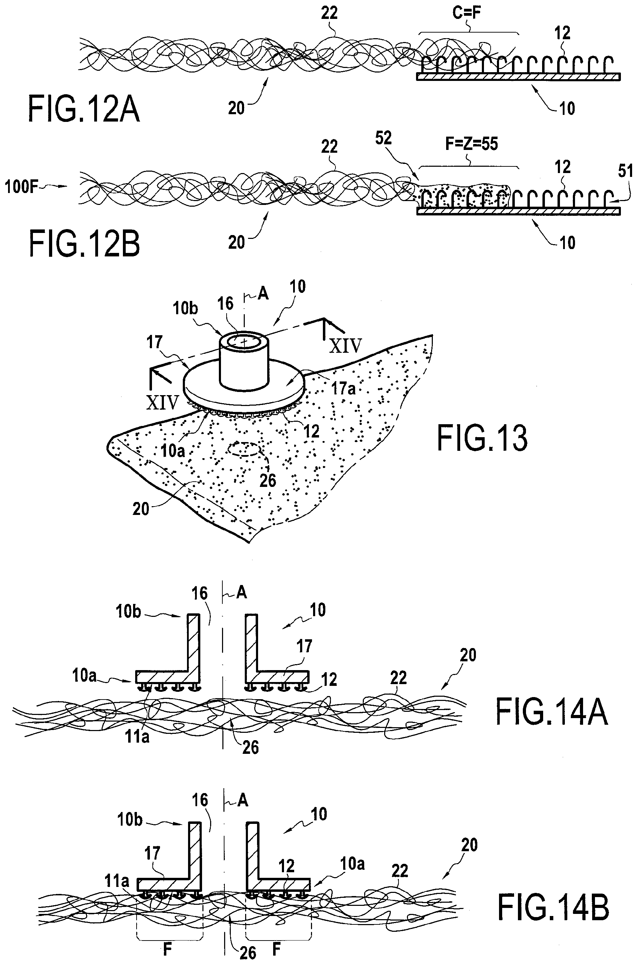

La figure 12A illustre le premier et le deuxième ensemble 10, 20 à l'issue de cette première étape. Les deux rubans 10, 20 sont maintenus en position l'un par rapport à l'autre grâce à la fixation auto-agrippante F, sans nécessité de moyens de guidage et de maintien supplémentaires.

Dans une deuxième étape illustrée par le tronçon T2, la fixation auto-agrippante F est traitée continûment dans la direction machine X.

Dans l'exemple, la zone de traitement Z s'étend sur l'ensemble de la zone de contact C, et le traitement est réalisé au moyen d'une molette M, notamment une molette chauffante, appliquant une pression sur les deux rubans 10, 20 pour déformer les crochets 12 du ruban de fixation 10 et/ou les fibres 22 du ruban support 20, en vue de solidariser les deux ensembles.

Le traitement peut aussi être réalisé sans chauffage, par pression uniquement, ou encore uniquement par chauffage, à distance notamment, ou encore par tout autre traitement adapté.

En fonction des matériaux choisis pour former le ruban de fixation 10 et le ruban support 20, et des conditions du traitement (température, pression appliquée, etc.), le traitement peut correspondre à l'un quelconque des exemples illustrés et décrits précédemment, notamment en liaison avec les figures 7A à 7C, 8 et 9. Les caractéristiques décrites en liaison avec ces exemples ne sont donc pas répétés ici.

De préférence, pour éviter une détérioration des crochets 12 présents sur la portion libre 18 du ruban de fixation 10, la mise en oeuvre du procédé sera cependant conforme à celle décrite en liaison avec la figure 8. Autrement dit : le ruban de fixation 10 est réalisé dans un matériau fusible à une température T2 supérieure à la température de fusion T1 du ruban support 20. La fixation auto-agrippante F réalisée entre les deux rubans 10, 20 est traitée en étant soumise à une température intermédiaire, supérieure à T1 mais inférieure à T2, de sorte que les fibres 22 fondent, et viennent enrober les crochets 12, restés, eux, intègres.

La structure assemblée ainsi obtenue 100F est illustrée sur la figure 12B. Elle comporte une première couche 51 constituée par le ruban de fixation 10 et comprenant des moyens de retenue pour une fixation auto-agrippante (ici les crochets 12, intacts, du ruban de fixation 10), et une deuxième couche 52, résultant de la déformation locale du ruban support 20, enrobant les moyens de retenue 12 sur une zone d'assemblage 55 correspondant à la zone de traitement pour les emprisonner et solidariser les dites couches.

Dans une troisième étape illustrée par le tronçon T3, la structure assemblée 100F est découpée dans une direction Y sensiblement transversale à la direction machine X, formant une pluralité de porte-crochets 100F' pour couches-culottes.

Le porte-crochets 100F' ainsi obtenu est équivalent à la structure assemblée 100F définie précédemment, dont il forme un fragment. Il comporte :
- une partie de fixation 74 (formée par la portion libre 18 de ruban de fixation 10) comprenant une base sensiblement plane délimitée par deux faces principales avant et arrière, et des crochets 12 faisant saillie depuis la face avant de ladite base,
- une partie support 72 en non-tissé (formée par la portion libre 28 de ruban support) située du côté avant de la base en s'étendant dans un plan intersectant les crochets 12, et
- entre la partie de fixation et la partie support (dans un plan intersectant le non-tissé d'une part et les crochets d'autre part), une zone d'assemblage.

Les figures 13 à 16 illustrent une autre application particulière du procédé selon le présent exposé, pour la fabrication d'une structure assemblée comprenant une base, notamment une nappe ou un produit composite, renforcée au voisinage d'un emplacement de perforation.

Une nappe ou bâche présente souvent des perforations destinées à recevoir des moyens de fixation tels que des sangles élastiques, des sandows, ou équivalents.

Une telle nappe est généralement renforcée, aux abords de ces perforations, par des inserts de renfort évidés généralement annulaires, du type oeillet. Ces oeillets sont habituellement fixés par clipsage, résultant en une déformation locale de la nappe et donc une baisse de résistance.

Dans d'autres applications, il est aussi nécessaire de perforer des éléments composites pour les fixer à leur support, risquant d'endommager ou d'affaiblir localement les dits éléments.

Le procédé d'assemblage selon le présent exposé constitue une solution avantageuse permettant de résoudre ce problème.

Sur la figure 13, on a représenté un premier ensemble 10 sous la forme d'un insert de renfort et un deuxième ensemble 20 formé par une nappe de fibres, ici un non-tissé.

L'insert de renfort 10 est destiné à être fixé à la nappe 20 au niveau d'un emplacement de perforation 26 tel qu'illustré schématiquement sur la figure 13, autrement dit un emplacement 26 de la nappe 20 déjà perforé ou (le plus souvent, et comme c'est le cas dans l'exemple) destiné à être perforé.

Dans l'exemple illustré, l'insert de renfort 10 présente une forme globalement cylindrique, définie autour d'un axe principal A, délimitant un trou traversant central 16.

L'insert de renfort 10 présente au moins une surface de jonction 11a s'étendant sensiblement orthogonalement à l'axe principal A, et dont fait saillie un champ d'éléments agrippants 12 adaptés à coopérer avec les fibres 22 de la nappe 20 afin de réaliser une fixation auto-agrippante F.

Dans l'exemple de la figure 12, la surface de jonction 11a est une face d'extrémité axiale de l'insert 10.

Plus particulièrement, des éléments agrippants 12 sont répartis sur toute ladite face d'extrémité 11a.

L'assemblage de l'insert de renfort 10 sur la nappe 20 est illustré plus en détail sur les figures 14A à 14D.

Dans une première étape illustrée par les figures 14A et 14B, l'insert de renfort 10 est amené au contact de la nappe 20 de sorte que les éléments agrippants 12 en saillie depuis sa surface de jonction 11a viennent coopérer avec les fibres 22 de la nappe 20 pour réaliser une fixation auto-agrippante F. Le trou 16 est placé sensiblement en regard de l'emplacement de perforation 26.

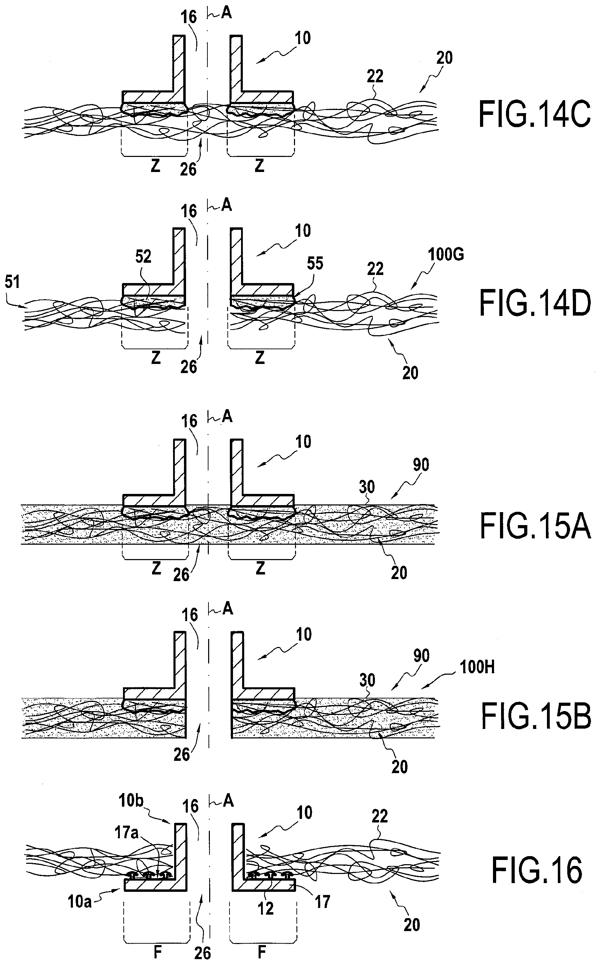

Dans une deuxième étape illustrée sur la figure 14C, la fixation auto-agrippante F est traitée. En fonction des matériaux choisis pour former la nappe 20 et les éléments agrippants 12 de l'insert de renfort 10, et des conditions du traitement (température, pression appliquée, etc.), le traitement peut correspondre à l'un quelconque des exemples illustrés et décrits précédemment, notamment en liaison avec les figures 7A à 7C, 8 et 9. Les caractéristiques décrites en liaison avec ces exemples ne sont donc pas répétés ici.

Dans l'exemple, les crochets 12 de l'insert 10 fondent sous l'effet du traitement, venant enrober les fibres 22 de la nappe 20, restées sensiblement intactes. La structure assemblée 100G comprenant la nappe 20 renforcée par l'insert de renfort 10 est illustrée sur la figure 14C. Elle comporte une première couche 51 constituée par la nappe 20 comprenant des moyens de retenue pour une fixation auto-agrippante (ici les fibres 22), et une deuxième couche, résultant de la déformation des crochets 12 de l'insert 10, enrobant les moyens de retenue 22 sur une zone d'assemblage 55.

Généralement, dans une troisième étape illustrée sur la figure 14D, la nappe 20 est alors perforée à l'emplacement de perforation 26, sensiblement selon l'axe principal de l'insert de renfort 10.

La nappe 20 est ainsi renforcée aux abords de son emplacement de perforation 26, sans pour autant avoir été endommagée ou déformée autour de l'insert de renfort 10. De plus, la fixation de l'insert 10 est sûre et définitive, garantissant la pérennité du produit.

L'exemple illustré n'est cependant pas limitatif.

Sur la figure 16, l'insert 10 présente une collerette externe 17 à l'une de ses extrémités axiales dite extrémité inférieure 10a, et sa surface de jonction, portant les éléments agrippants 12, est la face 17a de ladite collerette 17 orientée vers son extrémité supérieure 10b.

Comme illustré sur la figure, l'insert est alors inséré à travers un orifice pré-percé dans la nappe 20, de sorte que ladite surface de jonction 17a est amenée au contact de la nappe 20, pour réaliser la fixation auto-agrippante F qui assurera la solidarisation des deux ensembles.

Le procédé selon le présent exposé peut aussi être mis en oeuvre pour la fabrication d'un élément composite renforcé par au moins un insert de renfort.

Dans ce cas, les étapes initiales du procédé sont sensiblement identiques à celles décrites en liaison avec les figures 13 et 14A à 14C.

Pour former l'élément composite, dans une étape illustrée sur la figure 15A, la nappe 20 est associée à un troisième ensemble 30, ici une résine. La nappe est par exemple disposée dans un moule (non représenté) présentant la forme souhaitée pour l'élément composite, et imprégnée avec la résine 30, à l'intérieur du moule. La résine forme alors la matrice du composite, les fibres 22 de la nappe 20 formant ses moyens de renfort. La structure assemblée 100H comprenant le composite 90 renforcé par l'insert de renfort 10 est illustrée sur la figure 15A.

Le composite 90 ainsi obtenu peut ensuite être perforé au niveau de l'insert, sans risque de détérioration, comme illustré sur la figure 15B.

Selon une variante, l'assemblage du deuxième et du troisième ensemble 20, 30 peut aussi être réalisé en mettant en oeuvre le procédé selon le présent exposé, notamment ses exemples de mises en oeuvre décrits en liaison avec les figures 1 à 9.

Par exemple, la fabrication d'un élément composite renforcé par au moins un insert au niveau d'un emplacement de perforation s'apparente à l'exemple des figures 5A à 5C, le premier ensemble étant constitué par l'insert 10, le deuxième ensemble étant constitué par la nappe 20 et le troisième ensemble 30 comprenant une base muni d'un champ d'éléments agrippants adaptés à coopérer avec les fibres de la nappe 20.

Le premier et le deuxième ensemble forment une première fixation auto-agrippante au niveau de la surface de jonction de l'insert.

Le deuxième et le troisième ensemble forment une deuxième fixation auto-agrippante s'étendant de préférence sur toute leur surface de contact.

La première fixation auto-agrippante peut être traitée en premier, puis la deuxième, ou l'inverse. Les deux fixations auto-agrippantes peuvent encore être traitées concomitamment.

Finalement, le deuxième et le troisième ensemble forment un élément composite dont les moyens de renfort sont formés par les fibres de la nappe, restés intacts. Et le premier ensemble est solidarisé à l'élément composite, pour le renforcer au niveau de son emplacement de perforation.

## Revendications

1. Procédé dans lequel :
- on fournit un premier ensemble (10) comprenant un champ (13) d'éléments agrippants (12), notamment des crochets, et un deuxième ensemble (20) muni de moyens de retenue (22) adaptés à coopérer avec les éléments agrippants (12) du premier ensemble (10) pour réaliser une fixation auto-agrippante (F),
- on met le premier et le deuxième ensemble en contact de sorte que les éléments agrippants (12) du premier ensemble et les moyens de retenue (22) du deuxième ensemble réalisent une fixation auto-agrippante (F), et
- on traite une zone de traitement (Z) de la fixation auto-agrippante (F) pour déformer les éléments agrippants (12) du premier ensemble (10) et/ou les moyens de retenue (22) du deuxième ensemble (20), ce par quoi le premier et le deuxième ensemble (10, 20) sont solidarisés définitivement et forment ainsi une structure assemblée (100),
ledit procédé étant **caractérisé en ce que** les éléments agrippants (12) et/ou les moyens de retenue (22) sont réalisés dans un matériau thermoplastique .

2. Procédé selon la revendication 1, dans lequel le traitement est tel qu'après déformation, les éléments agrippants (12) ou les moyens de retenue (22) forment des moyens de renfort de la structure assemblée (100).

3. Procédé selon la revendication 1 ou 2, dans lequel le traitement est tel qu'après déformation, les éléments agrippants (12) ou les moyens de retenue (22) conservent une forme globalement inchangée.

4. Procédé selon l'une quelconque des revendications 1 à 3, dans lequel la zone de traitement (Z) forme une ligne continue.

5. Procédé selon l'une quelconque des revendications 1 à 4, dans lequel la zone de traitement (Z) représente au moins 50%, de préférence au moins 80%, de l'étendue totale de la fixation auto-agrippante (F).

6. Procédé selon l'une quelconque des revendications 1 à 5, dans lequel les moyens de retenue (22) comprennent des fibres.

7. Procédé selon l'une quelconque des revendications 1 à 6, dans lequel le traitement comprend l'application d'une pression sur la fixation auto-agrippante (F).

8. Procédé selon l'une quelconque des revendications 1 à 7, dans lequel le traitement comprend un traitement thermique.

9. Procédé selon l'une quelconque des revendications 1 à 8, dans lequel
- on fournit un troisième ensemble (30) comprenant un champ (33) d'éléments agrippants (32) adaptés à coopérer avec des moyens de retenue (22) du deuxième ensemble (20) pour réaliser une fixation auto-agrippante,
- on met le deuxième et le troisième ensemble (20, 30) en contact de sorte que le deuxième ensemble (20) est disposé entre le premier et le troisième ensemble (10, 30), et les éléments agrippants (32) du troisième ensemble (30) et les moyens de retenue (22) du deuxième ensemble (20) réalisent une deuxième fixation auto-agrippante (F2), et
- on traite une zone de traitement de la deuxième fixation auto-agrippante (F2) pour déformer les éléments agrippants (32) du troisième ensemble (30) et/ou les moyens de retenue (22) du deuxième ensemble (20), ce par quoi le deuxième et le troisième ensemble (20, 30) sont solidarisés définitivement.

10. Procédé selon la revendication 9, dans lequel les fixations auto-agrippantes réalisées entre le deuxième ensemble (20) et respectivement le premier et le troisième ensemble (10, 30) sont traitées concomitamment en une seule et même étape de traitement.

11. Procédé selon la revendication 9 ou 10, dans lequel, préalablement au traitement, les éléments agrippants (12) du premier ensemble (10) et les éléments agrippants (32) du troisième ensemble (30) sont amenés en coopération, les moyens de retenue (22) du deuxième ensemble (20) étant compris entre lesdits éléments agrippants (12, 32).

12. Procédé selon l'une quelconque des revendications 1 à 11, dans lequel le premier et le deuxième ensemble (10, 20) forment chacun une bande s'étendant longitudinalement, le premier et le deuxième ensemble (10, 20) sont superposés continument dans une direction longitudinale (X) sur au moins une zone de contact (C) sur laquelle les éléments agrippants (12) du premier ensemble (10) et les moyens de retenue (22) du deuxième ensemble (20) réalisent une fixation auto-agrippante (F), et ladite fixation auto-agrippante (F) est traitée continument dans la direction longitudinale (X) sur au moins une zone de traitement (Z).

13. Structure (100) comprenant au moins un premier ensemble (10) comprenant un champ (13) d'éléments agrippants (12) pour une fixation auto-agrippante (F) et au moins un deuxième ensemble (20) muni de moyens de retenue (22) coopérant avec les éléments agrippants (12) du premier ensemble (10) pour réaliser une fixation auto-agrippante (F), dans laquelle au moins une zone d'assemblage a été traitée de manière à déformer les éléments agrippants (12) du premier ensemble (10) et/ou les moyens de retenue (22) du deuxième ensemble (20) de sorte que le premier ensemble (10) et le deuxième ensemble (20) soient définitivement solidarisées l'une à l'autre, **caractérisée en ce que** lesdits moyens de retenue 22) sont réalisés dans un matériau thermoplastique.

14. Structure (100) selon la revendication 13, dans laquelle les moyens de retenue (12,22) comprennent des fibres.

15. Structure (100) selon l'une quelconque des revendications 13 à 14, dans laquelle la structure est un porte-crochets pour couche-culotte ou une base (20) renforcée à au moins un emplacement de perforation (26) par au moins un insert de renfort (10) muni d'un trou traversant (16).

16. Structure (100) selon l'une quelconque des revendications 13 à 15, dans laquelle les éléments agrippants présentent une hauteur totale, mesurée orthogonalement à la surface de la base dont ils sont issus, comprise entre 0,1 et 5 mm, de préférence entre 0,5 et 1,5 mm.

## Patentansprüche

1. Verfahren, bei dem:
- eine erste Baugruppe (10), die ein Feld (13) von Greifelementen (12) umfasst, insbesondere Haken, und eine zweite Baugruppe (20), die mit Haltemitteln (22) versehen ist, die dazu ausgelegt sind, mit den Greifelementen (12) der ersten Baugruppe (10) zusammenzuwirken, um eine selbstgreifende Verbindung (F) zu bilden, bereitgestellt werden,
- die erste und zweite Baugruppe so in Kontakt gebracht werden, dass die Greifelemente (12) der ersten Baugruppe und die Haltemittel (22) der zweiten Baugruppe eine selbstgreifende Verbindung (F) bilden, und
- eine Behandlungszone (Z) der selbstgreifenden Verbindung (F) behandelt wird, um die Greifelemente (12) der ersten Baugruppe (10) und/oder die Haltemittel (22) der zweiten Baugruppe (20) zu verformen, wodurch die erste und zweite Baugruppe (10, 20) dauerhaft miteinander verbunden werden und somit eine Verbundstruktur (100) bilden,
wobei das Verfahren **dadurch gekennzeichnet ist, dass** die Greifelemente (12) und/oder die Haltemittel (22) aus einem thermoplastischen Material hergestellt sind.

2. Verfahren gemäß Anspruch 1, wobei die Behandlung so erfolgt, dass die Greifelemente (12) oder Haltemittel (22) nach der Verformung Verstärkungsmittel für die Verbundstruktur (100) bilden.

3. Verfahren gemäß Anspruch 1 oder 2, wobei die Behandlung so erfolgt, dass die Greifelemente (12) oder Haltemittel (22) nach der Verformung eine im Wesentlichen unveränderte Form behalten.

4. Verfahren gemäß einem der Ansprüche 1 bis 3, wobei die Behandlungszone (Z) eine kontinuierliche Linie bildet.

5. Verfahren gemäß einem der Ansprüche 1 bis 4, wobei die Behandlungszone (Z) mindestens 50 %, vorzugsweise mindestens 80 % der Gesamtgröße der selbstgreifenden Verbindung (F) ausmacht.

6. Verfahren gemäß einem der Ansprüche 1 bis 5, wobei die Haltemittel (22) Fasern umfassen.

7. Verfahren gemäß einem der Ansprüche 1 bis 6, wobei die Behandlung das Aufbringen von Druck auf die selbstgreifende Verbindung (F) umfasst.

8. Verfahren gemäß einem der Ansprüche 1 bis 7, wobei die Behandlung eine Wärmebehandlung umfasst.

9. Verfahren gemäß einem der Ansprüche 1 bis 8, wobei
- eine dritte Baugruppe (30) bereitgestellt wird, die ein Feld (33) von Greifelementen (32) umfasst, die dazu ausgelegt sind, mit Haltemitteln (22) der zweiten Baugruppe (20) zusammenzuwirken, um eine selbstgreifende Verbindung zu bilden,
- die zweite und dritte Baugruppe (20, 30) so in Kontakt gebracht werden, dass die zweite Baugruppe (20) zwischen der ersten und dritten Baugruppe (10, 30) angeordnet ist, und die Greifelemente (32) der dritten Baugruppe (30) und die Haltemittel (22) der zweiten Baugruppe (20) eine zweite selbstgreifende Verbindung (F2) bilden, und
- eine Behandlungszone der zweiten selbstgreifenden Verbindung (F2) behandelt wird, um die Greifelemente (32) der dritten Baugruppe (30) und/oder die Haltemittel (22) der zweiten Baugruppe (20) zu verformen, wodurch die zweite und dritte Baugruppe (20, 30) dauerhaft miteinander verbunden werden.

10. Verfahren gemäß Anspruch 9, wobei die zwischen der zweiten Baugruppe (20) und der ersten bzw. dritten Baugruppe (10, 30) gebildeten selbstgreifenden Verbindungen in einem einzigen Behandlungsschritt gleichzeitig behandelt werden.

11. Verfahren gemäß Anspruch 9 oder 10, wobei vor der Behandlung die Greifelemente (12) der ersten Baugruppe (10) und die Greifelemente (32) der dritten Baugruppe (30) zusammenwirken gelassen werden, wobei die Haltemittel (22) der zweiten Baugruppe (20) zwischen den Greifelementen (12, 32) eingeschlossen sind.

12. Verfahren gemäß einem der Ansprüche 1 bis 11, wobei die erste und zweite Baugruppe (10, 20) jeweils ein sich in Längsrichtung erstreckendes Band bilden, die erste und zweite Baugruppe (10, 20) kontinuierlich in einer Längsrichtung (X) mindestens einer Kontaktzone (C) überlagert sind, auf der die Greifelemente (12) der ersten Baugruppe (10) und die Haltemittel (22) der zweiten Baugruppe (20) eine selbstgreifende Verbindung (F) bilden, und die selbstgreifende Verbindung (F) in der Längsrichtung (X) auf mindestens einer Behandlungszone (Z) kontinuierlich behandelt wird.

13. Struktur (100) mit mindestens einer ersten Baugruppe (10), die ein Feld (13) von Greifelementen (12) für eine selbstgreifende Verbindung (F) umfasst, und mindestens einer zweiten Baugruppe (20), die mit Haltemitteln (22) versehen ist, die mit den Greifelementen (12) der ersten Baugruppe (10) zusammenwirken, um eine selbstgreifende Verbindung (F) zu bilden, wobei mindestens eine Verbindungszone so behandelt wurde, dass die Greifelemente (12) der ersten Baugruppe (10) und/oder die Haltemittel (22) der zweiten Baugruppe (20) so verformt werden, dass die erste Baugruppe (10) und die zweite Baugruppe (20) dauerhaft miteinander verbunden werden, **dadurch gekennzeichnet, dass** die Haltemittel (22) aus einem thermoplastischen Material bestehen.

14. Struktur (100) gemäß Anspruch 13, wobei die Haltemittel (12, 22) Fasern umfassen.

15. Struktur (100) gemäß einem der Ansprüche 13 bis 14, wobei die Struktur ein Hakenhalter für Windeln oder eine Basis (20) ist, die an mindestens einer Perforationsstelle (26) durch mindestens einen Verstärkungseinsatz (10) verstärkt ist, der mit einem Durchgangsloch (16) versehen ist.

16. Struktur (100) gemäß einem der Ansprüche 13 bis 15, wobei die Greifelemente eine Gesamthöhe, gemessen orthogonal zur Oberfläche der Basis, von der sie abgehen, von zwischen 0,1 und 5 mm, vorzugsweise zwischen 0,5 und 1,5 mm aufweisen.

## Claims

1. A method comprising:
• providing a first unit (10) having a field (13) of grip elements (12), in particular hooks, and a second unit (20) provided with retention means (22) suitable for co-operating with the grip elements (12) of the first unit (10) in order to provide a self-gripping fastening (F);
• putting the first and second units into contact in such a manner that the grip elements (12) of the first unit and the retention means (22) of the second unit provide a self-gripping fastening (F); and
• treating a treatment zone (Z) of the self-gripping fastening (F) in order to deform the grip elements (12) of the first unit (10) and/or the retention means (22) of the second unit (20), whereby the first and second units (10, 20) are permanently secured to each other, thereby forming an assembled structure (100),
the method being **characterized in that** the grip elements (12) and/or the retention means (22) are made of thermoplastic material.

2. A method according to claim 1, wherein the treatment is such that after being deformed, the grip elements (12) or the retention means (22) form reinforcing means of the assembled structure (100).

3. A method according to claim 1 or claim 2, wherein the treatment is such that after being deformed, the grip elements (12) or the retention means (22) retain a shape that is generally unchanged.

4. A method according to any one of claims 1 to 3, wherein the treatment zone (Z) forms a continuous line.

5. A method according to any one of claims 1 to 4, wherein the treatment zone (Z) represents at least 50%, preferably at least 80% of the total extent of the self-gripping fastening (F).

6. A method according to any one of claims 1 to 5, wherein the retention means (22) comprise fibers.

7. A method according to any one of claims 1 to 6, wherein the treatment comprises applying pressure to the self-gripping fastening (F).

8. A method according to any one of claims 1 to 7, wherein the treatment comprises heat treatment.

9. A method according to any one of claims 1 to 8, comprising:
• providing a third unit (30) comprising a field (33) of grip elements (32) suitable for co-operating with the retention means (22) of the second unit (20) in order to provide a self-gripping fastening;
• putting the second and third units (20, 30) into contact so that the second unit (20) is arranged between the first and third units (10, 30), and the grip elements (32) of the third unit (30) and the retention means (22) of the second unit (20) provide a second self-gripping fastening (F2); and
• treating a treatment zone of the second self-gripping fastening (F2) to deform the grip elements (32) of the third unit (30) and/or the retention means (22) of the second unit (20), whereby the second and third units (20, 30) are permanently secured to each other.

10. A method according to claim 9, wherein the self-gripping fastenings provided between the second unit (20) and the first and third units (10, 30) respectively are treated simultaneously in a single treatment step.

11. A method according to claim 9 or claim 10, wherein prior to the treatment, the grip elements (12) of the first unit (10) and the grip elements (32) of the third unit (30) are put into co-operation, the retention means (22) of the second unit (20) lying between said grip elements (12, 32).

12. A method according to any one of claims 1 to 11, wherein the first and second units (10, 20) form respective longitudinally-extending strips, the first and second units (10, 20) being superposed continuously in a longitudinal direction (X) over at least one contact zone (C) where the grip element (12) of the first unit (10) and the retention means (22) of the second unit (20) provide a self-gripping fastening (F), and said self-gripping fastening (F) is treated continuously in the longitudinal direction (X) over at least one treatment zone (Z).

13. A structure (100) comprising at least a first assembly (10) comprisinga field (13) of grip elements (12) for a self-gripping fastening (F) and at least one second assembly (20) comprising retention means (22) co-operating with the grip elements (12) of the first assembly (10) to provide a self-gripping fastening (F); wherein over at least one assembly zone has been treated so as to deform the grip elements (12) of the first assembly (10) and/or the retention means (22) of the second assembly (20) so that the first assembly (10) and the second assembly (20) are permanently secured to each other , wherein the retention means (22) are made of thermoplastic material.

14. A structure (100) according to claim 13, wherein the retention means (12, 22) comprise fibers.

15. A structure (100) according to any one of claims 13 to 14, wherein the structure is a hook-carrier for diapers or a base (20) reinforced by at least one location (26) for a hole by means of at least one reinforcing insert (10) provided with a through bore (16).

16. A structure (100) according to any one of claims 13 to 15, wherein the grip elements comprise a total height along a direction orthogonal to the surface of the base from which they extend, comprised between 0.1 and 5 mm, preferably between 0.5 and 1.5 mm.
